(19) Europäisches Patentamt
European Patent Office
Office européen des brevets

(11) **EP 4 552 580 A2**

(12) **EUROPEAN PATENT APPLICATION**

(43) Date of publication:
**14.05.2025  Bulletin 2025/20**

(21) Application number: **24211131.8**

(22) Date of filing: **06.11.2024**

(51) International Patent Classification (IPC):
**A61B 8/00** *(2006.01)*  **G01S 15/89** *(2006.01)*
**A61B 34/20** *(2016.01)*  **A61B 90/00** *(2016.01)*

(52) Cooperative Patent Classification (CPC):
**A61B 8/4254; A61B 8/483; A61B 8/5215;**
**A61B 8/5253; A61B 8/5261; A61B 34/20;**
**A61B 90/37; G01S 15/89;** A61B 8/08; A61B 8/12;
A61B 8/429; A61B 8/4427; A61B 8/466; A61B 8/468

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **09.11.2023  US 202363597445 P**
**12.08.2024  US 202418800174**

(71) Applicant: **Siemens Medical Solutions USA, Inc.**
**Malvern, PA 19355 (US)**

(72) Inventors:
• **SHAH, Rishabh**
  **Wentworth Point, NSW, 2127 (AU)**
• **PETKOV, Kaloian**
  **Lawrenceville, NJ, 08648 (US)**

(74) Representative: **Horn Kleimann Waitzhofer**
**Schmid-Dreyer**
**Patent- und Rechtsanwälte PartG mbB**
**Theresienhöhe 12**
**80339 München (DE)**

(54) **SURGICAL GUIDANCE WITH COMPOUNDED ULTRASOUND IMAGING**

(57)    In one approach for surgical guidance with compounded ultrasound imaging, a neural field uses both probe tracking and ultrasound imaging to compound the ultrasound data into three-dimensions with alignment of the component fields of view. Accurate compounding is provided, and the compounding may operate in real-time. In another approach for visualization with pre-operative data, modeling (e.g., the neural field) from ultrasound is used to generate a 3D deformation field, which is then applied to the pre-operative data. The 3D deformation field may be applied in rendering rather than to the pre-operative volume dataset. This 3D deformation may be used in real-time, allowing for synchronized 3D ultrasound and 3D pre-operative imaging.

EP 4 552 580 A2

100 — Scanning by Ultrasound System

102 — Track Probe/FOV

110 — Compound into 3D Representation by Neural Field

120 — Render Image from 3D Representation

130 — Display Image

**FIG. 1**

**Description**

RELATED APPLICATION

The present patent document claims the benefit of the filing date under 35 U.S.C. § 119(e) of Provisional U.S. Patent Application Serial No. 63/597,445, filed November 9, 2023, which is hereby incorporated by reference.

BACKGROUND

The present embodiments relate to ultrasound-based image guidance for surgical procedures, e.g., laparoscopy. For example, anatomical structures are displayed from intra-operative internal tissue imaging. Image-based surgical guidance typically involves the display of images from 3D data derived from pre-operative imaging. This data augmentation during invasive surgery may employ tissue surface reconstruction algorithms to support the use of geometry-based measurements and improved depth cues during visualization. The accuracy of this data augmentation depends greatly on the type and quality of the patient imaging and its registration to the real world. Dynamic neural radiance fields (NeRF) have been used for deformable tissue reconstruction from stereo laparoscopic cameras, which handles complex surgical scenes better than the surface-based approaches. Separate neural radiance fields reconstructed during a procedure may further be fused in a single global field.

Three-dimensional (3D) ultrasound is beneficial for more complete visualization of the organs and internal structures (e.g., vessel trees in the liver), however the performance of the traditional approaches for compounding 2D ultrasound into 3D volumes do not scale well to real intra-operative imaging, resulting in artifacts in rendering and operator confusion for image guidance. Furthermore, since intraoperative ultrasound introduces volumetric deformations to the organs due to the required pressure from the ultrasound probe, it can be challenging to provide real-time synchronized views with pre-operative imaging and planning data.

Deformable or non-rigid registration between imaging modalities is used in a variety of clinical applications. Manual segmentation of the prostate gland in both pre-operative computed tomography (CT) and ultrasound, slice by slice, may be used for registration, but this does not provide for real-time performance and is subject to segmentation variability. Deep learning-based approaches achieve real-time performance only for 2D ultrasound to 3D CT registration. In another approach, segmented volume data is converted to surfaces (meshes), and deformation physics is simulated on the mesh data. A synthetic ultrasound image is generated from the meshes to produce a deformed ultrasound dataset for use in surgical simulators. This approach requires segmentation and deformation of the ultrasound data, which does not provide for image guidance from pre-operative imaging.

SUMMARY

Systems, methods, and non-transitory computer readable media are provided for surgical guidance with compounded ultrasound imaging. In one approach, a neural field uses both probe tracking and ultrasound imaging to compound the ultrasound data into three-dimensions with alignment of the component fields of view. Accurate compounding is provided, and the compounding may operate in real-time. In another approach for visualization with pre-operative data, modeling (e.g., the neural field) from ultrasound is used to generate a 3D deformation field, which is then applied to the pre-operative data. The 3D deformation field may be applied in rendering rather than to the pre-operative volume dataset. This 3D deformation may be used in real-time, allowing for synchronized 3D ultrasound and 3D pre-operative imaging.

In a first aspect, a method is provided for surgical guidance with compounded ultrasound imaging by an ultrasound system. The ultrasound system scans tissue of a patient, resulting in two-dimensional (2D) representations. Positions of the 2D representations are tracked. The 2D representations are compounded by input of the positions and 2D representations to a neural field. The neural field trained with a joint optimization of poses and parameters of the neural field. The compounding provides a 3D representation of the patient. A first image is rendered from the 3D representation and displayed.

In a second aspect, a medical system is provided for ultrasound compounding. An ultrasound probe tracker is configured to track an ultrasound probe during acquisition of ultrasound data. A memory is configured to store a machine-learned neural network formed as a neural field. An image processor is configured to compound the ultrasound data into a volume representation using the neural field. The ultrasound data as compounded has refined positional information from the ultrasound probe tracker. A display is configured to display an image from the volume representation.

In a third aspect, a method is provided for surgical guidance with compounded ultrasound imaging by an ultrasound system. The ultrasound system scans tissue of a patient, resulting in 2D representations. Pressures corresponding to the 2D representations are tracked. A model generates a 3D deformation field from the pressures and the 2D representations. In real-time with the scanning, a pre-operative image is rendered using the 3D deformation field and displayed. In embodiments, the third aspect can be considered an application of the first aspect.

The illustrative embodiments listed below summarize other features or aspects. Any one or more of the aspects described above or in the illustrative embodiments may be used alone or in combination with other of the illustrative embodiments, features, or aspects. Any aspects or features of one of method, system, or computer readable media may be used in the others of method, system, or computer readable media. These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

It is understood that the medical system of the second aspect may be implemented to carry out the methods or parts of the methods of the first and/or third aspect, e.g. as defined in the claims.

BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.

Figure 1 is a flow chart diagram of one implementation of a method for 3D compounding in ultrasound;

Figure 2 shows example ultrasound images from scanning and rendered images;

Figure 3 illustrates example compounding of 2D images into a 3D representation;

Figure 4 illustrates an example volume rendered segmentation image;

Figure 5 is a flow chart diagram of one implementation of a method for real-time deformation of pre-operative imaging based on modeling 3D ultrasound compounding;

Figure 6 illustrates example 3D deformation;

Figure 7 illustrates example visualization using 3D deformation in rendering of a pre-operative image;

Figure 8 is a block diagram of one implementation of a medical system for 3D compounding in ultrasound;

Figure 9 illustrates 3D compounding with deformation for image guidance in surgery; and

Figure 10 illustrates an example neural network.

DETAILED DESCRIPTION OF EMBODIMENTS

In one approach, surgical image guidance with neural 3D compounded ultrasound improves the anatomy field of view during interventional procedures compared to 2D ultrasound imaging. In another approach, synchronized viewing of 3D compounded ultrasound and pre-operative (e.g., CT planning) data is provided in real-time, such as based on real-time deformable volume rendering. The approaches may be used together, such as the neural field for compounding providing the 3D deformation used to deform the pre-operative imaging to reflect the current state of the tissue during surgery. In the examples used herein, the approaches are described for laparoscopy in the liver. For example, liver procedure guidance is provided with deformation-aware ultrasound compounding and visualization. The approaches or combination of approaches may be used in other interventions (e.g., open surgery or minimally invasive surgery) and/or for other organs (e.g., kidney).

In the deformation approach, 3D tissue deformation is modeled during the intra-operative compounding of 2D ultrasound images. The resulting deformation field improves the alignment of the pre-operative image (e.g., CT volume data) to the ultrasound for real-time image guidance during surgical procedures. The model-based deformation from the ultrasound compounding algorithm is leveraged to produce a 3D deformation field, which may be applied directly during volume rendering from pre-operative data. Techniques for direct visualization of the deformation displacement and time-based visualization of tissue changes are provided. The continuously updated deformation field is applied to real-time volume and surface visualization.

In one implementation, an end-to-end system enables rendering of deformed pre-operative datasets based on ultrasound captured during the procedures in real-time, allowing use during surgery. For example, the architecture provides for a real-time system from ultrasound compounding to rendering of deformed data with 128x128x128 volume compounding update rate at 10Hz and rendering update rate at 60Hz.

For the neural fields approach, a neural field models the 3D scene. A coordinate-based neural network is used in structure reconstruction and/or visualization rather than explicitly rendering primitives. The weights in the neural network implicitly represent the voxel data. Ultrasound compounding and anatomical structure reconstruction is based on a neural field. The neural network is initialized from 2D ultrasound slices using a meta-learning technique. The neural network is trained based on input of poses (e.g., tracked orientation) to refine the physical probe tracking data jointly with the compounding of 2D ultrasound data into a 3D volume dataset. Joint optimization of the image poses and network weights is used to refine the neural field representation.

Structure visualization (e.g., 3D segmentation) may be based on neural signed distance fields from the neural field. The neural field may learn a 3D signed distance function directly from 2D labeling or segmentation of 2D ultrasound images rather than using 3D segmentation.

In a further approach, the deformation field is learned during training. The neural field is trained to output the deformation field as well as or instead of 3D compounding the ultrasound data. The deformation field is learned directly during training, e.g., by incorporating differentiable deformable volume rendering.

In yet another approach, view optimization is based on differentiable direct volume rendering. Differentiable rendering models the explicit relationship between rendering parameters and resulting images in traditional image synthesis. Image-space derivatives are obtained with respect to the rendering parameters, which may be used in a variety of gradient-based optimization methods to solve inverse rendering problems or to compute the loss for training machine learning models directly in the space of rendered images.

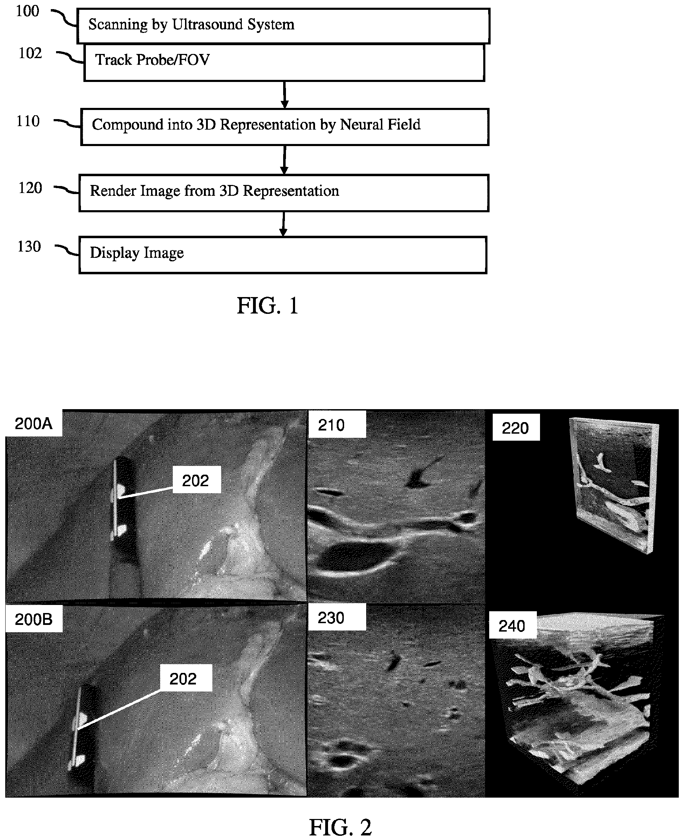

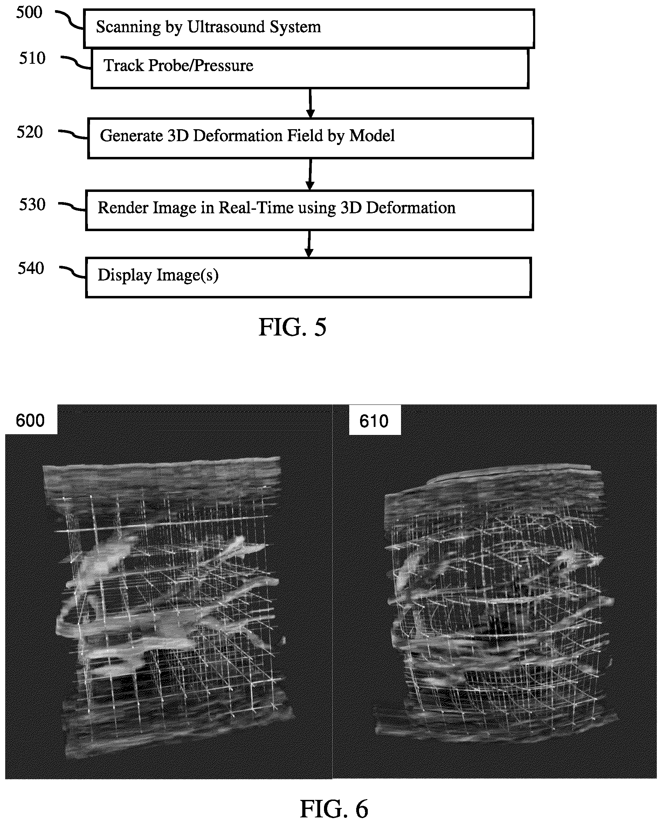

Figure 1 is directed to compounding for ultrasound imaging. Results from scanning a volume are compounded together based of field of view position during the scanning to form a volume representation. Figure 5 is directed to generating a deformation field so that a pre-operative volume dataset is rendered in a way reflecting, at least partially, the effect of pressure from the transducer on the organ of interest. A model is used to deform, such as the model used to compound.

Figure 1 is a flow chart diagram of one implementation of a method for surgical guidance with compounded ultrasound imaging by an ultrasound system. A neural field compounds in response to input of ultrasound data and probe or field of view tracking information. By machine training the neural field with joint optimization of the parameters of the neural field and probe or field of view position, the neural field accurately refines the position information and fills in missing information. The neural field representing the 3D scene is trained during image acquisition. In the context of ultrasound compounding, the network is initialized at the beginning of the first ultrasound probe sweep. The network may be pre-trained with procedure-specific and/or patient-specific data to speed up the training.

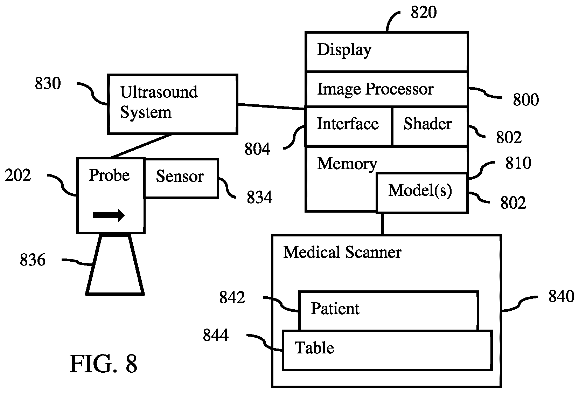

The method of Figure 1 is performed by a medical system, such as the medical scanner, ultrasound system, or medical system of Figure 8 or another medical system. For example, an ultrasound scanner acquires ultrasound data using a probe (transducer). A sensor and/or image processor tracks the probe or field of view. The image processor compounds (e.g., aligns and assembles) the ultrasound data from scanning at different times. The image processor and/or a graphics processing unit render an image from the compounded ultrasound data. A display displays the image. Other devices may perform any of the acts of Figure 1.

The method is performed in the order shown (e.g., top to bottom or numerical), but other orders may be used. For example, compounding in act 110 may occur interleaved with or between repetitions of acts 100 and 102. Acts 100 and 102 may be performed simultaneously.

Additional, different, or fewer acts may be provided. For example, acts 120 and 130 are not performed, such as where the compounding generates a deformation field used on pre-operative data without rendering and display for ultrasound data. As another example, acts for configuring the scanning, scanning by another modality (e.g., CT or magnetic resonance (MR)) and/or use of output (e.g., measurement) are performed.

In act 100, an ultrasound system scans tissue of a patient. The patient is scanned. The scanning results in multiple 2D representations, such as images formatted in a display domain (e.g., scan converted) or detected ultrasound data formatted in a scan domain rather than the display domain. In other embodiments, the 2D representations are acquired from memory or transfer over a computer network.

The 2D representations represent any part of the patient, such as representing an organ, head, or torso of the patient. For example, the 2D representations are from scanning a liver of the patient. Figure 2 illustrates an example. A probe 202 is positioned against an organ (e.g., liver) during surgery. A 2D scan is performed, resulting in a 2D image 210. Image 220 shows this image as a slab in a different orientation.

Different 2D representations represent a field of view at different positions within the organ of interest. For example, the transducer probe is translated, rocked, and/or rotated as a sequence of 2D representations are acquired. A volume of the organ is scanned, resulting in multiple 2D representations. In the example of Figure 2, the probe 202 is translated without rotation or with little rotation along the organ. At a different position, the image 230 is acquired. A collection of such images from different probe positions and/or fields of view within the organ are aligned and assembled (i.e., compounded) to form a volume representation 240.

The example of Figure 2 shows intra-operative scanning or scanning during open surgery. In alternative embodiments, the probe 202 is placed on the skin of the patient to scan internal organs, or the probe is scanned from within the body (e.g., transesophageal or intracardiac echocardiography). In yet other alternatives, a matrix array is used to directly scan a volume of the patient with little or no intended movement of the probe.

The scanning occurs while moving the transducer probe relative to the patient to scan the volume. During the scanning, the probe 202 is pressed against and may move along the tissue. This distorts or deforms the tissue, as well as adjacent tissues subjected to the pressure.

The 2D representations resulting from the scanning are ultrasound data or information derived therefrom. For example, different sample locations from scanning or pixel locations in imaging represent ultrasound intensity (e.g., B-mode information). Doppler (e.g., velocity, variance, and/or power) from ultrasound echoes may be provided instead or as well.

In another implementation, the ultrasound data is used to segment. The 2D representations are segmented to identify locations corresponding to anatomy or objects of interest. The locations or data are labeled, such as identifying vessels and/or tumors in the liver. The segmentation identifies a border and/or area corresponding to the object. The pixels or scan locations corresponding to the object are identified as the segmentation.

The segmentation is performed using any process or function, such as intensity thresholding with low pass filtering. In one implementation, full width, half maximum (FWHM) or intensity thresholding with various standard deviations is used to segment. Alternatively, a user (e.g., radiologist) or image processor manually segments. In another approach, a machine-learned model, such as an encoder-decoder-based neural network, outputs the segmentation or segmentations in response to input of the 2D representation. An image-to-image, U-Net, or encoder-decoder network trained to output the segmentation in response to input of the spatial 2D representation may be applied. The machine-trained model (segmentor) generates values for features in hidden layers in response to input of the medical image and uses the values of the features to output the segmentation.

In act 102, the positions of the probe and/or field of view during the scanning is tracked. The positions of the 2D representations relative to each other and/or another reference are determined. The probe or field of view is tracked in 3D sp ace.

Any now known or later developed tracking may be used. For example, a camera, probe detection, electromagnetic sensing, and/or data processing (e.g., correlation of 2D representations) tracks the position. In one implementation, fiducial markers on the probe are detected from a laparoscope video feed (e.g., the probe 202 is tracked from the camera images 200 as shown in Figure 2). Machine learning may be used to detect and track. Stereoscopic and/or depth cameras may improve the tracking performance. In another implementation, the probe is detected from intra-operative imaging by other (non-ultrasound) modalities, such as MR or fluoroscopy. In yet another implementation, the probe pose is detected via optical, electromagnetic, inertial, and/or other types of tracking. As another implementation, the ultrasound data from different 2D representations is correlated to determine the displacement, tracking the field of view.

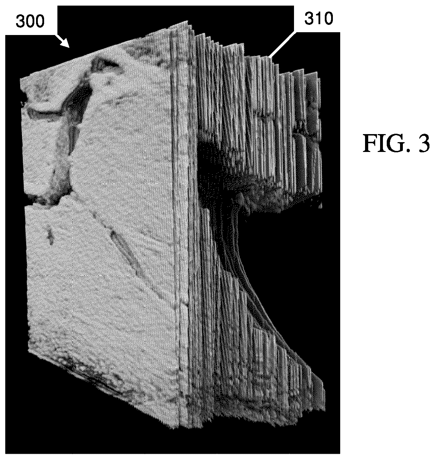

In act 110, the image processor compounds the 2D representations into a 3D representation. The 2D representations are aligned and assembled (e.g., stacked) to form the 3D representation. Figure 3 shows an example of the 2D representations as aligned from the tracking data. Multiple slices or 2D representations 310 are stacked to form the 3D representation 300.

In the example of Figure 3, the 3D representation may have gaps or irregularity due to the variance in speed of probe translation and/or pressure. The irregularity may result from or be made worse from inaccuracies in probe tracking.

To refine the tracking or 2D representation positions and/or to fill in any gaps to provide the 3D representation on a regular grid, the image processor applies a neural field for compounding. The tracked positions from act 102 and the 2D representations from act 100 are input the neural field. The neural field is a neural network arranged or with an architecture that is coordinate based, such as a 128x128x128 voxel-based arrangement of the neural network. Different nodes (e.g., weights or activation functions) represent different locations in three dimensions, providing a coordinate-based neural network for neural ultrasound compounding. The network architecture may use any positional encoding or activation functions that allow the network to fit high frequency signals, e.g., sinusoidal (e.g., SIREN), multiresolution hash or another positional encoding. The coordinate domain may be discretized using irregular or hierarchical grids to handle the varying complexity of the volume data due to the sparse acquisition. Other neural networks or machine learning models may be used to refine the position and/or compound. For example, an encoder or transformer outputs position in response to input of the position and 2D representation or positions and 2D representations.

The neural field is trained to output the 3D representation in response to input of the 2D representations and tracked positions. The machine-learned model may have been previously trained using training data of patient and/or procedure specific images. For a given patient, the neural field is further trained by optimization using new inputs. The model (e.g., neural field) is formed from an architecture (e.g., coordinate distribution) defining learnable parameters. For any pre-training, the training data includes many samples of inputs and corresponding ground truth (e.g., correct outputs) formed from a database of patient scans.

Meta-learning may be used to provide a domain and application-specific initialization of the network (e.g., different pre-training 2D representations may be used depending on the target organ, disease, and/or procedure from the same patient or a multitude of patients). A different neural field is provided for each of the different domains. Alternatively, the neural field is trained to compound for multiple or all domains.

Any training data for pre-training is gathered from medical records (images from the same patient or a cohort of patients that underwent the same or similar procedures (similarity according to a metric such as organ field of view or pathology). Alternatively, and/or additionally, simulation may be used to create the training data. The simulation may use physical objects, such as phantoms, and/or be based on computer modeling, such as using a physics model.

The neural field as initialized (e.g., from pretraining or initial imaging/tracking) is trained for compounding for a given patient. The training uses joint optimization of poses of the training images and parameters of the neural field (i.e., the learnable parameters). The 2D representations (e.g., images) from the tracked 2D ultrasound probe and their poses in 3D space are used as training input to the neural field that maps 3D positions to ultrasound intensities (e.g., Figure 3). In the optimization (e.g., Adam) used for training, the poses of the ultrasound images together with the parameters of the neural field are jointly optimized. The ultrasound probe tracking in application of the trained neural field is for pose initialization only, with the neural field compounding and refining the poses. This may reduce the impact of small tracking errors and tissue deformations during the scan. Alternatively, the image poses may be trained without initialization, to accommodate systems without probe tracking capabilities.

The training of the neural field uses rendering. The loss used in the optimization is between one or more renderings from the 3D representation output by the neural field and one or more of the input 2D images. The rendering is from the 3D representation to a 2D representation, such as extracting one or more slices or volume rendering to a view. The loss is based on or is a comparison of one or more renderings of the output with one of the input training images. The greater the difference, the greater the loss. The loss is used to alter values of one or more of the learnable parameters and the estimated poses in the joint optimization. The optimization continues until the loss is minimized for each sample set of training data.

Any rendering may be used, such as multiplanar image generation, volume rendering with ray casting or ray tracing, surface rendering, or another rendering. The rendering generates a view that is relatable or comparable to one or more of the 2D representations as input. In one implementation, differentiable rendering is used for 3D view optimization. Different renderings may be generated using different settings where each are compared to the input 2D representations. Alternatively, the differentiable rendering is performed to maximize or minimize (e.g., to optimize) a characteristic of the rendered view (e.g., visual entropy or occlusions). The optimized view is then compared to calculate the loss for machine training.

For training the machine learning model, the machine learning model arrangement (architecture) is defined. Any now known or later developed machine-learned model may be used. For example, an image-to-image network, U-Net, DenseNet, ResNet, or encoder-decoder network is used. Down sampling layers, convolutional layers, pooling layers, dropout layers, skip connections, up sampling layers, and/or other neural network layers may be used. The coordinate-based neural field is used in one implementation. Any architecture that receives image (spatial) information to output spatial (e.g., image) information may be used. The definition is by configuration or programming of the learning. The number of layers or units, type of learning, and other characteristics of the model are controlled by the programmer or user. In other embodiments, one or more aspects (e.g., number of nodes, number of layers or units, or type of learning) are defined and selected by the machine during the learning. Training data, including many samples of the input data and the corresponding output, is used to train. The relationship of the input to the output is machine learned.

The image processor or another processor machine trains the model (e.g., neural field). The training learns values for learnable parameters (e.g., weights, connections, filter kernels, values in activation functions, and/or other learnable parameters of the defined architecture). Deep or another machine learning may be used. The weights, connections, filter kernels, and/or other parameters are the features being learned. Using the training data, the values of the learnable parameters of the model are adjusted and tested to determine the values leading to an optimum estimation of the output given an input. Adam or another optimization is used to train.

During training, the loss is minimized. The loss function is a L1, L2, or other error function between the network output or renderings from the network output and the ground truth of the training data or the input data. Other losses may be used. Using optimization, different values of the learnable parameters are tested to minimize the loss (or maximize a reward).

The input to the network is a 3D position, and the output is a vector of parameters used by a volume renderer (attenuation, reflectance, scattering at the 3D position). Intensity or other measure may be output, which output may be converted to parameters used by the volume rendering. The vector provides information for a location, so different vectors are provided for different locations. The mapping from the input to the output uses a multilayer perceptron network architecture (MLP, fully connected neurons, nonlinear activation, e.g., ReLU). From the input image and its pose, the 3D location and final intensity of each pixel is known. The neural field is then trained to minimize the difference between the intensities in the training images and intensities produced by differentiable volume rendering with the attenuation, reflectance, scattering, etc. fields. A photometric loss function is used, but compounding can use a number of metrics to compare the training data images to the images synthesized from the neural fields, e.g., structural similarity index (SSIM). Once the network (neural field) is trained, the neural field can be used instead of voxel data in the algorithms of the visualization system (e.g., used for rendering).

The neural field compounds for that scan or surgery of the patient. The neural field may operate quickly to compound, allowing generation of the 3D representation in real-time.

In one implementation, the neural field or other model trains to provide the 3D representation as voxels representing ultrasound intensity or velocity distributed in three dimensions. 2D ultrasound with high in-plane resolution is input to output a 3D representation with high 3D resolution for B-mode or color mode ultrasound imaging.

In another implementation, the neural field or other model trains to provide the 3D representation as a 3D segmentation of an object or objects of interest. In training, the input may be the 2D representations or segmentations from the 2D representation and tracking, and the output is the 3D representation as a 3D segmentation.

For example, the 2D representations are segmented as signed distance fields. Each pixel or sample locations is given a value representing the distances to a nearest surface or location of the object of interest. The 3D representation is a signed distance field or function for the same object but in three dimensions. The compounding is into a 3D isolation mask, such as used for rendering. A neural signed distance field may be trained directly from object segmentation on the 2D ultrasound images as an alternative to performing the segmentation on voxel grids computed from inference on the fully trained ultrasound neural field. For example, the detected vessel contours are detected in the 2D ultrasound images, with 3D image poses refined from the joint optimization, resulting in a 3D point cloud which may then be used to infer the 3D signed distance function. Other segmentation representations than signed distance field or function may be used.

In another implementation, the neural field, neural network, machine-learned model, or another model is trained and used to output a deformation field. The scanning may deform tissue due to probe pressure. By inputting the tracking (e.g., pressure) and 2D representations, the 3D representation may be a field of values representing the magnitude and/or direction (e.g., vector) of deformation for each voxel. The deformation field is based on the scanning in real-time (e.g., with less than 0.1, 0.5, 1, or 3 seconds delay between scanning and generation of the deformation field) with the ultrasound scanning or imaging. The neural field or another model is trained or programmed using differentiable deformable volume rendering for calculating the loss. The model is trained to output the deformation field.

The ultrasound compounding may utilize 3D matrix probes. Since the volume scan by a matrix probe (e.g., 2D array for 3D scanning) takes time, the sample locations may shift due to patient and/or sonographer movement. The compounding by the neural field or other model may refine the position information to remove distortions caused by the motion. In other implementations, x-ray, CT, MR, or other non-ultrasound imaging modalities are used instead of ultrasound. A differentiable direct volume renderer is used to train the voxel representation from X-ray or other modality projected images.

In act 120, the image processor and/or a graphics processing unit renders an image from the 3D representation output by the model. Surface rendering, ray casting, ray tracing, or another type rendering from a volume dataset to the 2D image domain may be used. The rendering or renderings from the 3D representation may be for augmented reality, virtual reality, and/or display on a screen. The 3D representation is rendered to pixels or locations in two dimensions.

The rendering generates an image. The image may be updated as the scanning updates. For real-time viewing, the image is updated to reflect the current state currently represented by the ultrasound scanning. For example, a B-mode image representing a volume is rendered from ultrasound intensities of the 3D representation.

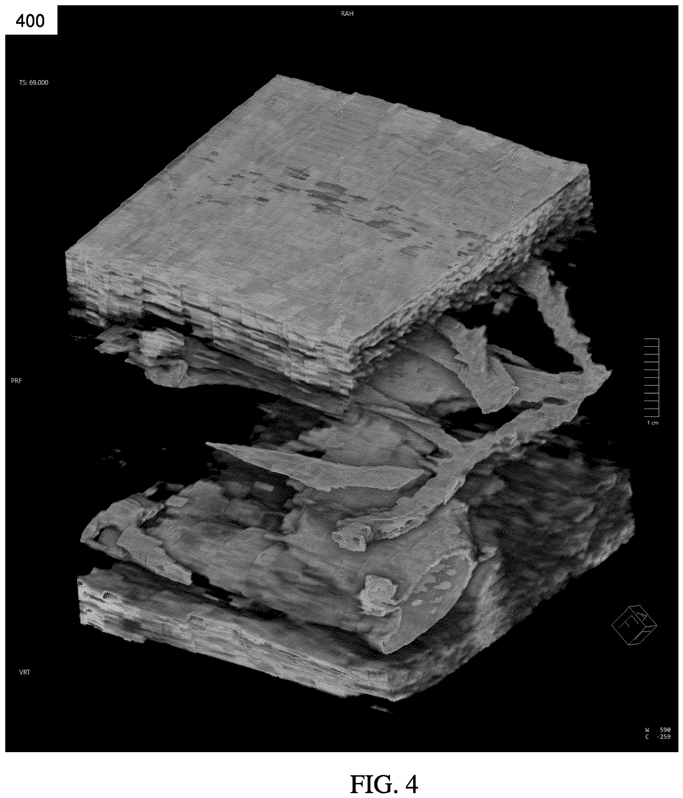

In one implementation, the rendering uses voxel classification by a transfer function to depict a 3D representations of an object (segmentation). For example, vessels and/or tumors in a liver are rendered. The 3D representation is of voxels labeled as belonging to vessels and/or tumors. The rendered image shows the object without showing or with reduced emphasis on other objects. Figure 2 shows an example rendering of the compounded ultrasound with a transfer function emphasizing the liver blood vessels 220 at the beginning of the ultrasound sweep and 240 at the end of the ultrasound sweep. The 3D rendering is aligned with the direction of the ultrasound probe sweep. Figure 4 shows another example rendering 400 of a view from a different view direction relative to the volume of the blood vessels in the liver.

In one implementation, the image processor or graphics processing unit samples the neural field directly for rendering. The compounded neural field representation of the ultrasound data is in or can be converted to a regular grid by inference on the voxel locations and visualized with traditional volume rendering techniques. In this implementation, the neural field is sampled directly, leveraging the compact and data-adaptive neural network representation for memory efficient processing. The same applies to neural signed distance fields trained directly from structures in the input images - traditional surface extraction may be performed on voxel-grid reconstruction of the neural field SDF, or the neural field SDF may be sampled directly in a ray-casting or sphere-tracing algorithm.

In another implementation, the rendering uses differentiable rendering. The rendering is repeated in an optimization to maximize or minimize a characteristic of the rendered image. One or more variables for rendering are altered to result in the desired view from the 3D representation. The visualization system may use a differentiable renderer to compute optimized views for the segmented structures, e.g., when displaying the augmented guidance visuals on a separate display in the operating room. The differentiable renderer may operate on the segmented object surfaces, directly on the volume data, on selected objects in the scene, such as the liver vessel trees, and/or the entire 3D scene. In general, the renderer computes image-space derivatives for various rendering parameters, such as the camera orientation, and then gradient descent or other optimization techniques are used to compute a change in the rendering parameter(s) that maximizes an objective function (e.g., visual entropy (i.e., the amount of visual information in the image)). Different application-specific image metrics may be used, e.g., penalizing the occlusion of important structures or the overlapping of vessel structures. Machine learning-based image quality metrics may be used, e.g., trained on rankings of views by clinical experts. The optimized view may then be used in rendering, such as used by a photorealistic renderer (e.g., volumetric Monte Carlo path tracing). The view optimization may be initialized using a laparoscopy or other camera view.

The rendering may augment a laparoscope camera view. View synthesis from the neural field may produce images for Augmented Reality headsets or multi-view autostereoscopic displays.

In other approach, the 3D representation includes or is a deformation field. The deformation field is applied to a volume dataset of another modality (e.g., pre-operative CT or MR). The rendering is from the other volume dataset as deformed. Figure 5 below is directed to this approach.

Combinations of implementations may be provided. For example, the same or different neural field is trained to output multiple types of information, such as output a 3D representation of ultrasound intensity, a 3D representation of segmentation, and/or a 3D deformation field. One or more images are rendered using any combination of output information.

In act 130, one or more images are displayed on a display device. The image is presented to a viewer on a screen or printout. Different images may be presented, such as a multi-planar reconstruction showing three orthogonal views and a volume rendering in four quadrants. A segmentation image may be displayed. The image may be of the deformation field to inform the physician of changes due to scanning. The image may be or include rendering or imaging from pre-operative scanning or planning. Different renderings, renderings from different views, 2D images with or without 3D images, 3D images with or without 2D images, images from different types of rendering, stereoscopic views, augmented reality views, and/or other imaging may be provided.

Figure 5 shows a flow chart of one implementation of a method for surgical guidance with compounded ultrasound imaging by a medical system. Deformed pre-operative datasets are rendered based on ultrasound captured during a surgical procedure in real-time, which allows use during surgery. Real-time deformation is derived from modeling of ultrasound acquisition, and then the deformation is applied to the pre-operative dataset. For example, 128x128x128 volume compounding (see Figure 1) provides the deformation in 3D, which is then applied to the pre-operative dataset for rendering or as part of rendering. The ultrasound compounded volume update may be provided at 10Hz during ultrasound acquisition with asynchronous visualization system updates provided at 60Hz, e.g., during user interaction.

The method of Figure 5 is performed by a medical system, such as the medical scanner, ultrasound system, or medical system of Figure 8 or another medical system. For example, an ultrasound scanner acquires ultrasound data using a probe (transducer). A sensor and/or image processor tracks the probe or field of view. The image processor generates the 3D deformation field with a model. The image processor and/or a graphics processing unit renders an image in real-time with the scanning using the 3D deformation field. A display displays the image. Other devices may perform any of the acts of Figure 1.

The method is performed in the order shown (e.g., top to bottom or numerical), but other orders may be used. For example, generating in act 520 may occur interleaved with or between repetitions of acts 500 and 510. Acts 500 and 510 may be performed simultaneously.

Additional, different, or fewer acts may be provided. For example, acts 530 and/or 540 are not performed, such as where the deformation field is used for measurement. As another example, acts for configuring the scanning, imaging by ultrasound and/or another use of the output are performed.

In act 500, the ultrasound system scans the tissue of the patient. The same or different scanning used in act 100 of Figure 1 is performed. The scan results 2D representations.

In act 510, the pressure from the probe on the tissue is tracked. The pressure may be assumed to be one-dimensional, such as directed along a normal from the face of the transducer or probe. Alternatively, the pressure in two or three-dimensions is tracked.

In one implementation, the position of the probe or field of view is used as the pressure. The difference in position from placement against the tissue to when the tissue is deformed due to pressing after placement represents the pressure. In other implementations, the actual pressure is measured, such as with a strain gauge sensor. Any probe tracking where the measurement correlates with pressure may be used, such as tracking position or pressure sensing.

In act 520, the image processor generates a 3D deformation field using a model. The model uses the pressures and 2D representations to generate the 3D deformation field.

Any model may be used. For example, a physics or bio-mechanical model is used to determine the deformation. Using a fitting or optimization, the 2D representations and pressures are used to determine the deformation of the tissue at different sample locations (e.g., voxels).

In one implementation, deformations in 2D are determined. Image processing or a machine-learned model determines deformation in 2D. The 2D deformations are then determined in 3D by compounding the ultrasound-based 2D representations. In another implementation, the image processor generates an approximate 3D deformation field from simplified 2D deformation by compounding a vector field in parallel to the compounding for ultrasound imaging. Both the 3D vector field and the 3D ultrasound representation are registered to the pre-operative planning using computational approaches, such as machine-learning-based approaches, and physical tracking approaches.

In another implementation, a neural network, such as a neural field, generates the 3D deformation field. For example, and as discussed above for Figure 1, a neural field is trained to generate a 3D deformation field from input of 2D representations and pressure or tracking. The same or different neural field may compound to form a 3D representation for ultrasound intensity and/or segmentation.

Figure 6 shows an example. The image 600 shows the volume in a regular grid reflected by parallel lines. The image 610 shows the volume and grid rendered with a deformation field applied. The lines are shifted corresponding to node or intersections being shifted. Each node or voxel has a shift representing the deformation.

The 3D deformation field is generated in real-time with the scanning (e.g., less than 0.1, 0.5, 1, or 3 seconds from completion of image scanning for the 2D representations to generation of the 3D deformation field). For real-time imaging, the 2D representations (e.g., 2D ultrasound images), 3D representation (e.g., ultrasound volume dataset of intensities), and 3D deformation field are streamed to a renderer or graphics processing unit through a streaming interface. A standard interface, such as OpenIGTLink, or custom optimized interface for low latency applications may be used. The interface may further implement compression, such as video codecs, differentiable compression schemes, wavelet-based compression, or a machine learning-based approach such as neural volumes. The interface is configured to synchronize the data for rendering to visualize in real-time. Computational components (e.g., image processor and graphics processing unit) may be coupled or linked for higher performance at the cost of deployment flexibility. For example, a graphics processing unit-based ultrasound compounding implementation may use backward projection directly into the GPU memory of a rendering service.

In act 530, the image processor and/or graphics processing unit renders a pre-operative image (image based on pre-operative dataset) in real-time with the scanning of act 500 using the 3D deformation field. The primary target is operative ultrasound for surgical guidance and/or pre-operative CT or other data deformed to match the ultrasound data or current tissue state. One or more images rendered from the pre-operative data reflects the current deformation as detected from the ultrasound data. Alternatively, the ultrasound data (e.g., 3D representation) is deformed to a state corresponding to the pre-operative scan (i.e., transducer pressure is countered) by applying the inverse of the computed 3D deformation field for fusion with the planning (pre-operative) data.

For deforming the pre-operative dataset as part of rendering, the 3D deformation is used as an indexing texture in the rendering pipeline of a graphics processing unit. The 3D deformation volume is used as an indexing texture to displace the voxels of the target volume (e.g., CT dataset or pre-operative data) while sampling. This deformation volume is updated in real-time based on the streaming data from 3D compounding, so the sampling is updated. The 3D deformation field is a separate volume used in indexing in the rendering pipeline to render from the pre-operative dataset. The resulting rendered image from the pre-operative dataset includes the deformation.

In another implementation, the graphics processing unit computes data sampling locations from splines generated from the 3D deformation field. Parametric descriptions of the deformation field, such as splines or other curves, are computed. The parametric representation (e.g., splines) of various orders are evaluated during rendering to compute data sampling locations. As an optimization, a 3D deformation volume may be computed from the parametric representation in a preprocessing step, such as computing in a compute shader of the graphics processing unit or pipeline. This deformation volume is then used during rendering to select sample locations in the pre-operative dataset to render an image from the pre-operative dataset.

As another implementation, the graphics processing unit deforms the pre-operative volume representation in the compute shader based on the deformation field. The pre-operative volume representation is rendered from the deformed pre-operative volume representation. In a pre-processing (pre-rendering) act, the pre-operative volume is deformed in the compute shader. The deformed volume is then sampled without any additional computations during rendering and may be streamed to pipeline components that do not support the deformation volume, e.g., image processing or segmentation. This compute shader execution occurs every time the deformation data is streamed in from compounding.

Other rendering-based application of the 3D deformation to render the pre-operative dataset may be used. As an alternative, the image processor deforms the pre-operative dataset (e.g., volume) prior to providing to the graphics processing unit. The 3D deformation field is applied to the pre-operative dataset. The graphics processing unit then renders from the deformed data set.

In act 540, the display displays the rendered pre-operative image. The displayed image includes at least a portion reflecting the deformation caused by the ultrasound probe. By rendering in real-time with the ultrasound scanning, the rendered pre-operative image reflects, at least in part, the alteration or change in position of the tissue due to the probe pressure.

The image may be of the image rendered from the pre-operative dataset alone. Alternatively, the pre-operative image is displayed with an ultrasound image. The images from different modalities may be synchronized. Since modelling deformation is used with rendering using the deformation, real-time imaging of the pre-operative dataset with deformation to account for probe pressure is provided. The ultrasound images are generated in real-time, allowing synchronizing the pre-operative image to the ultrasound image. The current state of the tissue deformation is reflected in both types of images to guide the surgeon for easy comparison.

Various combinations of images, including deformed and not deformed and including pre-operative and ultrasound, may be displayed. For example, both operative ultrasound and pre-operative planning data are visualized together for surgical guidance. In one implementation, one volume image is overlaid over the other. The ultrasound image is rendered with different classification in the same scene as the pre-operative (e.g., CT) volume or composited with different opacity or shading. This allows quick comparison between the two at a glance.

In another implementation, the reference (ultrasound) volume is rendered as a silhouette on an image rendered from a fully shaded deformed volume (the deformed pre-operative volume). The datasets may be visualized together, where the pre-operative (e.g., CT) image is rendered with full shading and the ultrasound image is rendered as a silhouette to provide the additional context without crowding the image with redundant and low detail information.

As another implementation, the magnitude of displacement from the 3D deformation is used as a metric for shading, thus giving a heatmap of amount of change at each voxel in the volume. The deformation can be applied to 3D volume, MPR, and/or segmentation mesh rendering. Changes in meshes from deformations can be animated to highlight the changes in structures in areas of interest. For example, liver vessels may change over time, and these changes can be highlighted using the deformation data.

The images may be of segmented objects. The segmentations may be overlaid on an image of the region to highlight particular structure or displayed alone.

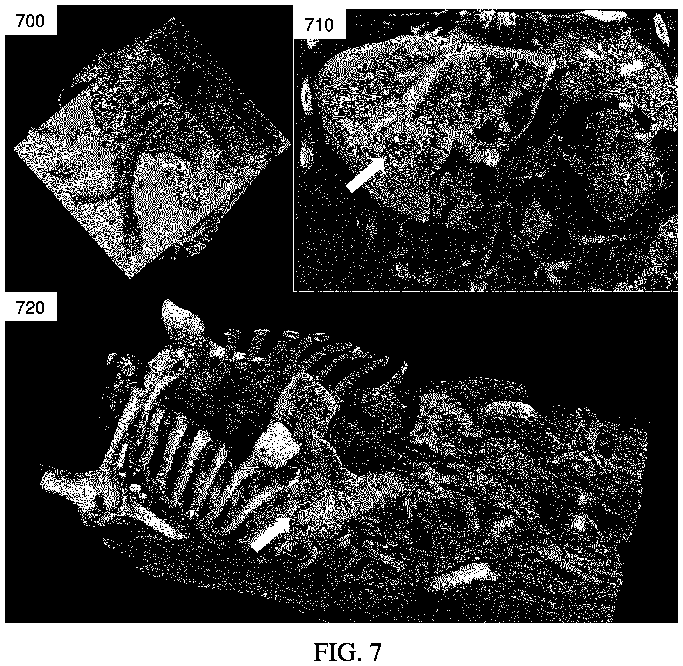

Figure 7 shows an example display. Image 700 is an ultrasound Multiplanar Reformation (MPR) image from the ultrasound compounding volume depth-embedded in the 3D volume rendering of the ultrasound compounding volume. Image 710 contains a 3D volume rendering with embedded MPR of a pre-operative CT from the same patient as Image 700, with the ultrasound field of view indicated by an embedded box indicator at the white arrow.. The pre-operative volume is deformed as part of rendering to match the deformation of tissue reflected in the image 710. Image 720 is a rendering of the pre-operative volume of a larger portion of the patient with or without deformation. The field of view of the ultrasound image 700 is visualized as an embedded box indicator at the arrow.

In another implementation, a multi-planar reconstruction is displayed. The deformation is applied to render the planar reconstructions from the pre-operative data. The deformation is not applied to render from 3D representation (e.g., volume) of the pre-operative dataset.

Figure 8 shows one embodiment of a medical system for ultrasound compounding and/or deformation. The medical system includes the image processor 800, the memory 810, the display 820, the ultrasound system 830 and/or the medical scanner 840. The display 820, image processor 800, and/or memory 810 may be part of the medical scanner 840 (e.g., CT scanner), ultrasound system 830, computer, server, workstation, or another system for image processing medical images from a scan of a patient. A workstation or computer without the medical scanner 840 and/or ultrasound system 830 may be used as the medical system.

Additional, different, or fewer components may be provided. For example, a computer network is included for remote image generation of locally captured image data. The machine-learned models 802 are applied as standalone applications on the workstation or a local device or as a service deployed on network (cloud) architecture. In another example, the medical scanner 840 is not provided, such as where the pre-operative dataset was previously acquired and is stored in the memory 810.

The ultrasound system 830 is a diagnostic medical ultrasound scanner. The ultrasound system 830 includes a probe 202 for scanning a field of view 836. An optional sensor 834 is provided on the probe 202 or for sensing the probe 202. The ultrasound system 830 may include a transmit beamformer, receive beamformer, B-mode detector, color flow or Doppler detector, filters, scan converter, and/or other components.

The probe 202 is an ultrasound transducer with an array, such as a one-dimensional array, of transducer elements. The probe 202 is sized, shaped, and made of material for handheld or robotic use externally to the patient, such as for pressing against the skin of the patient to scan. Alternatively, the probe 202 is sized, shaped, and made of material for use in an orifice (e.g., trans-esophageal), for insertion into the patient (e.g., intra-operative probe or intracardiac catheter), or for intra-operative use. Other probes 202 may be used.

The ultrasound system 830 uses the probe 202 to scan the field of view 836. A scan provides a 2D representation. The scanning is repeated to acquire multiple 2D representations. As the probe 202 is shifted or moved, the field of view 836 shifts or moves to scan other planes in the tissue of the patient.

The sensor 834 mounts to the probe 202 and/or is mounted to sense the probe 202. The sensor 834 may be a camera, electromagnetic sensor, accelerometer, strain gauge, pressure sensor, and/or another sensor to detect probe prose. In another approach, the medical scanner 840 is the sensor, detecting the probe 202 or fiducials on the probe 202 while the probe 202 is within or adjacent to the patient. In an alternative, the image processor 800 compares (e.g., correlates with different shifts) 2D representations in a sequence to determine the change in position or pose. The sensor 834 is an ultrasound probe tracker configured to track the ultrasound probe 202 during acquisition of ultrasound data (e.g., during 2D scanning of a plane).

The image processor 800 or another processor may operate with the sensor for tracking. Any image processing (e.g., fiducial recognition and position determination) is performed by the processor to track.

In one implementation, the image processor 800 or another processor performs segmentation or other process on the ultrasound data. For example, the ultrasound data is converted into a 2D field of signed distances for and object for each of the 2D representations.

The medical scanner 840 is a diagnostic or therapeutic scanner (e.g., CT, x-ray, or MR scanner). The scanner 840 operates pursuant to one or more settings to scan a patient 842 resting on a bed or table 844. The settings control scanning including transmission, reception, reconstruction, and image processing. A scanning protocol is followed to generate data representing the patient 842. The patient 842 is imaged by the scanner 840 using the settings. The scanner 840 generates the pre-operative or other planning dataset, such as a volumetric representation of the patient 842 at a time prior to the scanning by the ultrasound system 830.

The ultrasound data (e.g., 2D representations such as ultrasound images), segmentations (e.g., 2D signed distance fields), pre-operative data, compounded 3D representation, 3D deformation field, machine learned model 802, values of learned parameters, probe tracking data, rendered images, and/or other information are stored in a non-transitory computer readable memory, such as the memory 810. The memory 810 is configured to store the machine-learned neural network (e.g., model 802) formed as a neural field. The neural field was trained with joint optimization of probe pose and parameters of the neural field. Other models 802 may be stored, such as for different domains (e.g., liver verses kidney imaging).

The memory 810 is an external storage device, RAM, ROM, database, and/or a local memory (e.g., solid state drive or hard drive). The same or different non-transitory computer readable media may be used for the instructions and other data. The memory 810 may be implemented using a database management system (DBMS) and residing on a memory, such as a hard disk, RAM, or removable media. Alternatively, the memory 810 is internal to the processor 800 (e.g., cache).

The instructions for implementing the training or application processes, the methods, and/or the techniques discussed herein are provided on non-transitory computer-readable storage media or memories, such as a cache, buffer, RAM, removable media, hard drive, or other computer readable storage media (e.g., the memory 810). Non-transitory computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code, and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the manner in which the present embodiments are programmed.

The image processor 800 is a controller, control processor, general processor, micro-processor, tensor processor, digital signal processor, three-dimensional data processor, graphics processing unit, application specific integrated circuit, field programmable gate array, artificial intelligence processor, digital circuit, analog circuit, combinations thereof, or another now known or later developed device for processing image data. The image processor 800 is a single device, a plurality of devices, or a network of devices. For more than one device, parallel or sequential division of processing may be used. Different devices making up the image processor 800 may perform different functions. In one implementation, the image processor 800 is a control processor or other processor of the medical scanner 840 or ultrasound system 830. The image processor 800 operates pursuant to and is configured by stored instructions, hardware, and/or firmware to perform various acts described herein.

The image processor 800 may include an interface 804. The interface 804 is configured to receive tracking data, ultrasound data, and/or pre-operative data at different rates and/or enforce synchronization. The interface 804 may be a standard or custom interface for receiving data and/or transmitting (e.g., outputting) data, such as images to the display 820 for visualization.

The image processor 800 includes a shade compute (shader 802) rasterization (rasterizer), and/or parallel processors forming a rendering pipeline. Alternatively, other hardware components or just the processor is provided.

The image processor 800 or another remote processor is configured to train machine learning architectures, such as model 802. For example, the neural field is trained using training data. As another example, the neural field is trained using joint optimization to compound from probe tracking and images for a patient. The machine-learning model 802 is trained to generate the 3D representation, such as a compounding or 3D deformation field.

Alternatively, or additionally, the image processor 800 is configured to apply the machine-learned model(s) 802. The machine-learned models 802 may include a machine-learned network for 2D segmentation.

The image processor 800 is configured to compound the ultrasound data into a volume representation using the neural field (model 802). The ultrasound data as compounded has refined positional information from the ultrasound probe tracker or sensor 834. The compounding forms the 3D representation or representations from ultrasound data (2D or 3D) from the ultrasound system 830 and tracking from the sensor 834. The 3D representation is ultrasound intensities, a segmented object in 3D, and/or a 3D deformation field. For example, the model 802 used to compound ultrasound data also generates a 3D deformation field. As another example, the image processor 800 is configured to generate a 3D signed distance field or another segmentation of an object in response to input of 2D segmentations or 2D ultrasound data to the model 802. In another example, a different model (computational and/or machine-learned model 802) generates the 3D deformation field from the ultrasound scans.

The image processor 800 is configured to generate an output, such as an image showing the segmentation. The image processor 800, using the shader 802 or another rendering component(s), renders one or more images. The images may be rendered from the 3D compounded ultrasound data. Alternatively, or additionally, the images are rendered from pre-operative data. The rendered image from the pre-operative data may reflect tissue as deformed based on rendering using the 3D deformation field. Real-time imaging may be provided. As another alternative, or addition, the image processor 800 is configured to generate a visualization of the segmentation (contour). An image is rendered from a 3D segmentation.

The display 820 is a CRT, LCD, projector, plasma, printer, tablet, smart phone or another now known or later developed display device for displaying the output, such as one or more rendered images from a volume representation. A sequence of images rendered during the surgery may be displayed to guide the surgeon. For example, pre-operative images may be rendered in real-time with the ultrasound scanning based on a sequence of deformation fields generated from the ultrasound scanning (e.g., from the model 802 used to compound or another model 802). As another example, an ultrasound and/or pre-operative dataset image is rendered as a 3D segmentation using a signed distance field generated by the compounding model 802.

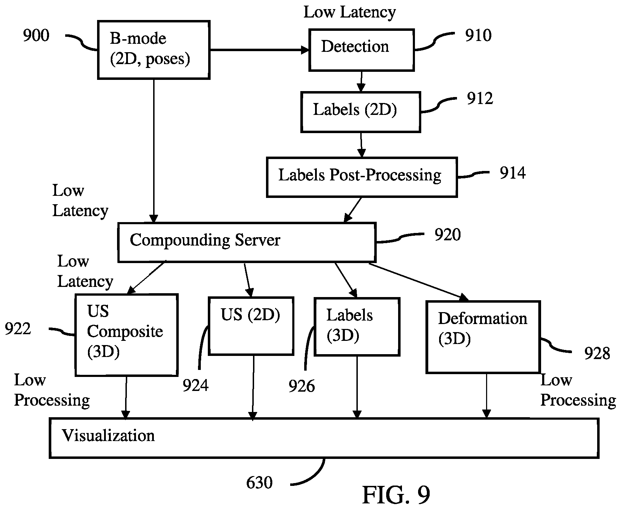

Figure 9 is a block diagram illustrating another example implementation of a system for visualization using modeling (e.g., a neural field) to compound and/or rendering at least one image using 3D deformation. Ultrasound B-mode 2D representations and probe poses 900 are provided to a processor for detection 910 at a low latency and to the compounding server 920 at the low latency. The detection 910 generates segmented or labeled 2D representations 912. The labeled representations 912 may be further processed, such as filtered or separated (e.g., different objects distinguished) 914. The compounding server 920 uses a neural field, neural fields, and/or other models to generate the 3D representations (e.g., ultrasound composite or compounded 3D representation 922, labeled or segmented 3D representation 926, and/or deformation field 3D representation 928. The server 920 may pass or also generate 2D ultrasound representations. The various representations are generated with the same or different latency. The visualization 630 is a graphics processing unit or other image generator for generating images from any one, combination, or all of the representations 922, 924, 926, and/or 928. The image generation may require different amounts of processing.

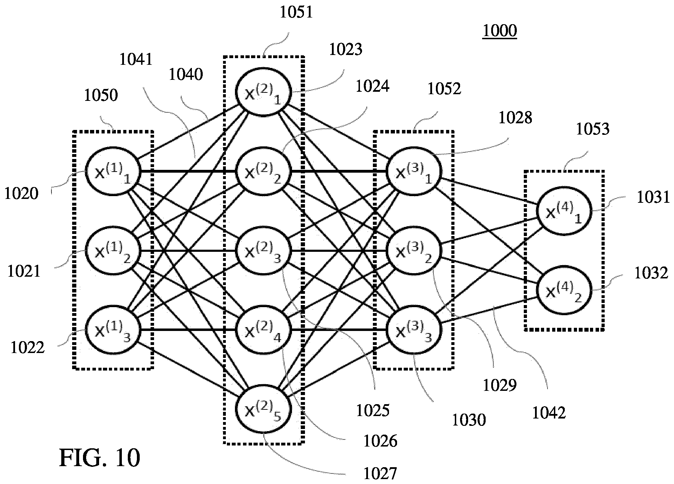

Figure 10 shows an embodiment of an artificial neural network 1000, in accordance with one or more embodiments. Alternative terms for "artificial neural network" are "neural network," "artificial neural net," or "neural net." The artificial neural network 1000 may be used in part in, for example, the one or more machine learning based networks utilized for compounding or generating 3D deformation. The artificial neural network 1000 may be arranged or designed as a neural field for ultrasound 3D compounding and/or generating a deformation field.

The artificial neural network 1000 includes nodes 1020-1032 and edges 1040-1042, wherein each edge 1040-1042 is a directed connection from a first node 1020-1032 to a second node 1020-1032. In general, the first node 1020-1032 and the second node 1020-1032 are different nodes 1020-1032, it is also possible that the first node 1020-1032 and the second node 1020-1032 are identical. For example, in Figure 10, the edge 1040 is a directed connection from the node 1020 to the node 1023, and the edge 1041 is a directed connection from the node 1021 to the node 1023. An edge 1040-1042 from a first node 1020-1032 to a second node 1020-1032 is also denoted as "ingoing edge" for the second node 1020-1032 and as "outgoing edge" for the first node 1020-1032.

In this embodiment, the nodes 1020-1032 of the artificial neural network 1000 may be arranged in layers 1050-1053, wherein the layers may include an intrinsic order introduced by the edges 1040-1042 between the nodes 1020-1032. In particular, edges 1040-1042 may exist only between neighboring layers of nodes. In the embodiment shown in Figure 5, there is an input layer 1050 including only nodes 1020-1022 without an incoming edge, an output layer 1053 including only nodes 1031 and 1032 without outgoing edges, and hidden layers 1051, 1052 in-between the input layer 1050 and the output layer 1053. In general, the number of hidden layers 1051, 1052 may be chosen arbitrarily. The number of nodes 1020-1022 within the input layer 1050 usually relates to the number of input values of the neural network 1000, and the number of nodes 1031-1032 within the output layer 1053 usually relates to the number of output values of the neural network 1000. For a neural field, the number of layers and nodes corresponds to the coordinate system. Each node may be connected to adjacent nodes in any direction in a bi-directional edge. The input may be to all the nodes with output to all the nodes.

In one approach, the network architecture is an MLP with a small number of fully-connected layers, where the input to the network is a 3D position (mapped to a higher dimension using positional encoding), and the output of the network is a vector of parameters used by a volume renderer (attenuation, reflectance, scattering at the 3D position).

A (real) number may be assigned as a value to every node 1020-1032 of the neural network 1000. Here, $x^{(n)}_i$ denotes the value of the i-th node 1020-1032 of the n-th layer 1050-1053. The values of the nodes 1020-1022 of the input layer 1050 are equivalent to the input values of the neural network 1000, the value of the nodes 1031-1032 of the output layer 1053 is equivalent to the output value of the neural network 1000. Furthermore, each edge 1040-1042 may include a weight being a real number, in particular, the weight is a real number within the interval [-1, 1] or within the interval [0, 1]. Here, $w^{(m,n)}_{i,j}$ denotes the weight of the edge between the i-th node 1020-1032 of the m-th layer 1050-1053 and the j-th node 1020-1032 of the n-th layer 1050-1053. Furthermore, the abbreviation $w^{(n)}_{i,j}$ is defined for the weight $w^{(n,n+1)}_{i,j}$.

In particular, to calculate the output values of the neural network 1000, the input values are propagated through the neural network. In particular, the values of the nodes 1020-1032 of the (n+1)-th layer 1050-1053 may be calculated based on the values of the nodes 1020-1032 of the n-th layer 1050-1053 by:

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise or through adjacent nodes through the neural network 1000, wherein values of the input layer 1050 are given by the input of the neural network 1000, wherein values of the first hidden layer 1051 may be calculated based on the values of the input layer 1050 of the neural network 1000, wherein values of the second hidden layer 1052 may be calculated based in the values of the first hidden layer 1051, etc.

In order to set the values $w^{(m,n)}_{i,j}$ for the edges, the neural network 1000 has to be trained using training data. In particular, training data includes training input data and training output data (denoted as $t_i$). For a training step, the neural network 1000 is applied to the training input data to generate calculated output data. In particular, the training data and the calculated output data include a number of values, said number being equal with the number of nodes of the output layer.

In particular, a comparison between the calculated output data and the training data is used to recursively adapt the weights within the neural network 1000 (backpropagation algorithm). In particular, the weights are changed according to:

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ may be recursively calculated as:

$$\delta^{(n)}_j = \left(\sum_k \delta^{(n+1)}_k \cdot w^{(n+1)}_{j,k}\right) \cdot f\left(\sum_i x^{(n)}_i \cdot w^{(n)}_{i,j}\right)$$

based on $\delta^{(n+1)}_j$, if the (n+1)-th layer is not the output layer, and

$$\delta^{(n)}_j = \left(x^{(n+1)}_k - t^{(n+1)}_j\right) \cdot f\left(\sum_i x^{(n)}_i \cdot w^{(n)}_{i,j}\right)$$

if the (n+1)-th layer is the output layer 1053, wherein f is the first derivative of the activation function, and $y^{(n+1)}_j$ is the comparison training value for the j-th node of the output layer 1053.

The following is a list of non-limiting Illustrative Embodiments disclosed herein. Illustrative Embodiments for one set or type (e.g., method or system) may be provided in or combined with other sets of types of Illustrative Embodiments.

Illustrative Embodiment 1: A method for surgical guidance with compounded ultrasound imaging by an ultrasound system, the method comprising: scanning, by the ultrasound system, tissue of a patient, the scanning resulting in two-dimensional (2D) representations; tracking positions of the 2D representations; compounding the 2D representations by input of the positions and 2D representations to a neural field, the neural field trained with a joint optimization of poses and parameters of the neural field, the compounding providing a three-dimensional (3D) representation of the patient; rendering a first image from the 3D representation; and displaying the first image.

Illustrative Embodiment 2: The method of Illustrative Embodiment 1, wherein tracking comprises tracking with a camera, probe detection, or electromagnetic sensing.

Illustrative Embodiment 3: The method of any of Illustrative Embodiments 1-2, wherein scanning comprises scanning while moving a transducer probe relative to the patient.

Illustrative Embodiment 4: The method of any of Illustrative Embodiments 1-3, wherein the 2D representations comprise images of ultrasound intensity, wherein compounding comprises providing the 3D representation as voxels representing intensity distribution in three dimensions, and wherein rendering comprises rendering the first image as ultrasound intensities as a function of location in two dimensions.

Illustrative Embodiment 5: The method of any of Illustrative Embodiments 1-4, wherein the 2D representations comprise 2D segmentations of an object, wherein compounding comprises providing, by the neural field, a signed distance field representing a 3D segmentation of the object as the 3D representation, and wherein rendering the first image comprises rendering the first image from the 3D segmentation of the object.

Illustrative Embodiment 6: The method of any of Illustrative Embodiments 1-5, wherein compounding comprises compounding by the neural field, the neural field trained with a loss based on comparison of a rendering of an output with one of the 2D representations.

Illustrative Embodiment 7: The method of any of Illustrative Embodiments 1-6, wherein compounding comprises compounding by the neural field, the neural field comprising a coordinate-based neural network.

Illustrative Embodiment 8: The method of Illustrative Embodiment 7, wherein compounding comprises compounding where the coordinate-based neural network comprises sinusoidal, multiresolution hash or another positional encoding.

Illustrative Embodiment 9: The method of any of Illustrative Embodiments 1-8, wherein rendering comprises sampling the neural field directly.

Illustrative Embodiment 10: The method of any of Illustrative Embodiments 1-9, wherein rendering comprises view optimization with differentiable rendering.

Illustrative Embodiment 11: The method of any of Illustrative Embodiments 1-10, wherein compounding comprises providing the 3D representation as a deformation field based on the scanning as real-time ultrasound, and wherein rendering comprises rendering a pre-operative image based on the deformation field.

Illustrative Embodiment 12: The method of Illustrative Embodiment 11, wherein compounding comprises compounding with the neural field, the neural field trained using differentiable deformable volume rendering.

Illustrative Embodiment 13: A medical system for ultrasound compounding, the medical system comprising: an ultrasound probe tracker configured to track an ultrasound probe during acquisition of ultrasound data; a memory configured to store a machine learning neural network formed as a neural field; an image processor configured to compound the ultrasound data into a volume representation using training of the neural field as a joint optimization of probe pose and parameters of the neural field, the ultrasound data as compounded having refined positional information from the ultrasound probe tracker; and a display configured to display an image from the volume representation.

Illustrative Embodiment 14: The system of Illustrative Embodiment 13, wherein the volume representation comprises a deformation field, and wherein the image comprises a pre-operative image rendered using the deformation field.

Illustrative Embodiment 15: The system of any of Illustrative Embodiments 13-14, wherein the ultrasound data comprises two-dimensional fields of signed distances for an object, wherein the volume representation comprises a signed distance field for the object, and wherein the image comprises a three-dimensional segmentation rendered using the signed distance field.

Illustrative Embodiment 16: A method for surgical guidance with compounded ultrasound imaging by an ultrasound system, the method comprising: scanning, by the ultrasound system, tissue of a patient, the scanning resulting in two-dimensional (2D) representations; tracking pressures corresponding to the 2D representations; generating a three-dimensional (3D) deformation field by a model from the pressures and the 2D representations; rendering, in real-time with the scanning, a pre-operative image using the 3D deformation field; and displaying the pre-operative image.

Illustrative Embodiment 17: The method of Illustrative Embodiment 16, wherein generating comprises generating by the model comprising a neural field.

Illustrative Embodiment 18: The method of any of Illustrative Embodiments 16-17, wherein rendering comprises using the deformation field as an indexing texture.

Illustrative Embodiment 19: The method of any of Illustrative Embodiments 16-18, wherein rendering comprises computing data sampling locations from splines generated from the deformation field.

Illustrative Embodiment 20: The method of any of Illustrative Embodiments 16-19, wherein rendering comprises deforming a pre-operative volume representation based on the deformation field in a compute shader and rendering from the deformed pre-operative volume representation.

Illustrative Embodiment 21: The method of any of Illustrative Embodiments 16-20, wherein displaying comprises displaying the pre-operative image synchronized with an ultrasound image from the scanning.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope of the invention.

## Claims

1. A method for surgical guidance with compounded ultrasound imaging by an ultrasound system (830), the method comprising:

    scanning (100), by the ultrasound system (830), tissue of a patient, the scanning (100) resulting in two-dimensional (2D) representations;
    tracking (102) positions of the 2D representations;
    compounding (110) the 2D representations by input of the positions and 2D representations to a neural field (802), the neural field (802) trained with a joint optimization of poses and parameters of the neural field (802), the compounding (110) providing a three-dimensional (3D) representation of the patient;
    rendering (120) a first image from the 3D representation; and
    displaying (130) the first image.

2. The method of claim 1, wherein tracking (102) comprises tracking (102) with a camera, probe detection, or electromagnetic sensing.

3. The method of claim 1 or 2, wherein scanning (100) comprises scanning (100) while moving a transducer probe relative to the patient.

4. The method of any one of claims 1-3, wherein the 2D representations comprise images of ultrasound intensity, and wherein compounding (110) comprises providing the 3D representation as voxels representing intensity distribution in three dimensions, and wherein rendering (120) comprises rendering (120) the first image as ultrasound intensities as a function of location in two dimensions; and/or wherein the 2D representations comprise 2D segmentations of an object, and wherein compounding (110) comprises providing, by the neural field (802), a signed distance field representing a 3D segmentation of the object as the 3D representation, and wherein rendering (120) the first image comprises rendering (120) the first image from the 3D segmentation of the object.

5. The method of any one of claims 1-4, wherein compounding (110) comprises compounding (110) by the neural field (802), the neural field (802) having been trained with a loss based on comparison of a rendering (120) of an output with one of the 2D representations; and/or wherein compounding (110) comprises compounding (110) by the neural field (802), the neural field (802) comprising a coordinate-based neural network, the coordinate-based neural network, in particular, comprising sinusoidal or multiresolution hash positional encoding.

6. The method of any one of claims 1-5, wherein rendering (120) comprises sampling the neural field (802) directly; and/or wherein rendering (120) comprises view optimization with differentiable rendering (120).

7. The method of any one of claims 1-6, wherein compounding (110) comprises providing the 3D representation as a deformation field based on the scanning (100) as real-time ultrasound, and wherein rendering (120) comprises rendering (120) a pre-operative image based on the deformation field; and, in particular, wherein compounding (110) comprises compounding (110) with the neural field (802), the neural field (802) trained using differentiable deformable volume rendering (120).

8. A medical system for ultrasound compounding (110), the medical system comprising:

    an ultrasound probe tracker configured to track an ultrasound probe during acquisition of ultrasound data;

    a memory configured to store a machine-learned neural network formed as a neural field (802);

    an image processor configured to compound the ultrasound data into a volume representation using training the neural field (802) as a joint optimization of probe pose and parameters of the neural field (802), the ultrasound data as compounded having refined positional information from the ultrasound probe tracker; and
    a display configured to display an image from the volume representation.

9. The system of claim 8, wherein the volume representation comprises a deformation field, and wherein the image comprises a pre-operative image rendered using the deformation field.

10. The system of claim 8 or 9, wherein the ultrasound data comprises two-dimensional fields of signed distances for an object, wherein the volume representation comprises a signed distance field for the object, and wherein the image comprises a three-dimensional segmentation rendered using the signed distance field.

11. A method for surgical guidance with compounded ultrasound imaging by a medical system, the method comprising:

scanning (100), by the ultrasound system (830), tissue of a patient, the scanning (100) resulting in two-dimensional (2D) representations;
tracking (102) pressures corresponding to the 2D representations;
generating a three-dimensional (3D) deformation field by a model from the pressures and the 2D representations;
rendering (120), in real-time with the scanning (100), a pre-operative image using the 3D deformation field; and
displaying (130) the pre-operative image.

12. The method of claim 11, wherein generating comprises generating by the model comprising a neural field (802), in particular, the neural field (802) of the compounding steps according to the method of any one of claims 1 - 7.

13. The method of claim 10 or 11, wherein rendering (120) comprises at least one of:

using the deformation field as an indexing texture;
computing data sampling locations from splines generated from the deformation field; and/or
deforming a pre-operative volume representation based on the deformation field in a compute shader and rendering (120) from the deformed pre-operative volume representation.

14. The method of any one of the preceding claims, wherein displaying (130) comprises displaying (130) the pre-operative image and/or the first image synchronized with an ultrasound image from the scanning (100).

15. The system of any one of claims 8 - 10, wherein the system is implemented to carry out the method of any one of the preceding claims 1 - 7 or 11 - 14, wherein the image processor is, in particular, implemented to carry out the compounding steps according to the method of any one of claims 1 - 7, the ultrasound data including the two-dimensional representations and/or the positions, and the three-dimensional (3D) representations being the volume representations.

FIG. 1

FIG. 2

FIG. 3

FIG. 4

| 500 | Scanning by Ultrasound System |
| 510 | Track Probe/Pressure |

| 520 | Generate 3D Deformation Field by Model |

| 530 | Render Image in Real-Time using 3D Deformation |

| 540 | Display Image(s) |

FIG. 5

FIG. 6

FIG. 7

820

Display

Image Processor

Interface | Shader

Memory

Model(s)

800

802

810

802

830

Ultrasound
System

804

202

Probe | Sensor

834

836

Medical Scanner

Patient

Table

842

844

840

FIG. 8

900 ⌇ B-mode (2D, poses)

Low Latency

Detection ⌇ 910

Labels (2D) ⌇ 912

Labels Post-Processing ⌇ 914

Low Latency

Compounding Server ⌇ 920

Low Latency

922 ⌇ US Composite (3D)

US (2D)

924

Labels (3D)

926

Deformation (3D) ⌇ 928

Low Processing

Low Processing

Visualization

630 ⌇

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63597445